(19) Europäisches Patentamt
European Patent Office
Office européen des brevets

(11) **EP 3 588 074 A2**

(12) **EUROPÄISCHE PATENTANMELDUNG**

(43) Veröffentlichungstag:
01.01.2020 Patentblatt 2020/01

(51) Int Cl.:
**G01N 27/38** (2006.01)  **G01N 27/416** (2006.01)
**G01N 27/404** (2006.01)  **G01N 27/44** (2006.01)

(21) Anmeldenummer: **19177266.4**

(22) Anmeldetag: **29.05.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **07.06.2018 DE 102018113640**

(71) Anmelder: **ProMinent GmbH**
**69123 Heidelberg (DE)**

(72) Erfinder:
• **Koppert, Klaus**
**68239 Mannheim (DE)**
• **Schopf, Holger**
**74074 Heilbronn (DE)**
• **Winkler, Thomas**
**76149 Karlsruhe (DE)**

(74) Vertreter: **WSL Patentanwälte Partnerschaft mbB**
**Kaiser-Friedrich-Ring 98**
**65185 Wiesbaden (DE)**

(54) **VERFAHREN ZUR REINIGUNG, KONDITIONIERUNG, KALIBRATION UND/ODER JUSTAGE EINES AMPEROMETRISCHEN SENSORS**

(57) Um ein Verfahren zur Reinigung, Konditionierung, Kalibration, Justage und Konditionierung eines amperometrischen Sensors einer Messeinrichtung zur Bestimmung eines Inhaltsstoffes in einem Elektrolyten mit einer durch eine selektiv durchlässige Membran verschlossenen, den Elektrolyten enthaltenden Messkammer, in der die Arbeitselektrode und eine mit der Arbeitselektrode elektrisch verbundene Referenzelektrode angeordnet ist, wobei die Membran für den zu bestimmenden Inhaltsstoff durchlässig ist, wobei die Bestimmung des Inhaltsstoffes während eines Messintervalls dadurch erfolgt, das eine Spannung zwischen Arbeitselektrode und Referenzelektrode angelegt wird, der Strom, der über die elektrische Verbindung von Arbeitselektrode und Referenzelektrode fließt, gemessen wird und aus dem gemessenen Strom auf den Inhaltsstoff rückgeschlossen wird, bereitzustellen, das eine vergleichbare oder sogar eine bessere Reinigungs- und Konditionierwirkung als die aus dem Stand der Technik bekannten Verfahren aufweist, für das jedoch ein einfacherer apparativer Aufbau ausreichend ist, wird ein Verfahren beschrieben, das dadurch gekennzeichnet ist, dass ein Konditioniermittel in der Messeinrichtung erzeugt wird, wobei als Konditioniermittel entweder ein Oxidationsmittel, welches an der Arbeitselektrode reduziert wird, oder ein Reduktionsmittel, welches an der Arbeitselektrode oxidiert wird, verwendet wird.

**Figur 1**

EP 3 588 074 A2

**Beschreibung**

**Gegenstand der Erfindung**

Die Erfindung betrifft ein Verfahren zur Reinigung, Konditionierung, Kalibration und/oder Justage eines amperometrischen Sensors einer Messeinrichtung zur Bestimmung eines Inhaltsstoffes in einer Probe, der eine durch eine selektiv durchlässige Membran verschlossene, einen Elektrolyten enthaltende Messkammer aufweist, in der die Arbeitselektrode und eine mit der Arbeitselektrode elektrisch verbundene Arbeitsreferenzelektrode angeordnet sind, wobei die Membran für den zu bestimmenden Inhaltsstoff durchlässig ist. Die Bestimmung des Inhaltsstoffes erfolgt während eines Messintervalls dadurch, dass eine Spannung zwischen Arbeitselektrode und Arbeitsreferenzelektrode angelegt wird, der Strom, der über die elektrische Verbindung von Arbeitselektrode und Arbeitsreferenzelektrode fließt, gemessen wird und aus dem gemessenen Strom auf den Inhaltsstoff rückgeschlossen wird. Die Erfindung betrifft auch einen amperometrischen Sensor, eine Messeinrichtung zur Durchführung des erfindungsgemäßen Verfahrens und die Verwendung eines Konditioniermittels.

**Hintergrund der Erfindung**

Das Ergebnis elektrochemischer Messverfahren zur Bestimmung von Inhaltsstoffen in einer Probe ist oft stark abhängig von der aktiven Elektrodenoberfläche eines Sensors. Allerdings führt der Betrieb über die Zeit zu Ablagerungen und Veränderungen auf der Elektrode, die die aktive Elektrodenoberfläche verringern und dadurch das Ergebnis des Messverfahrens ungewollt beeinträchtigen. Dies führt in der Praxis zu einem hohen Reinigungs- und Justageaufwand und es besteht die Gefahr unsicherer und falscher Messwerte.

Aus diesem Grund ist eine Reinigung der Elektrode in regelmäßigen Zeitabständen notwendig. Hierzu sind verschiedene Verfahren aus dem Stand der Technik bekannt.

Zunächst einmal gibt es die Möglichkeit der mechanischen Reinigung, beispielsweise durch Abrieb mit Sand, Korund oder ähnlich harten Materialien. Dieses Verfahren ist jedoch aufwändig und kostenintensiv und sorgt bei Messeinrichtungen für schmutzbelastete Proben meist für unzuverlässige Messwerte. Weiterhin muss die Apparatur zumindest für einen Wechsel des Abriebmaterials auseinander gebaut werden.

Eine weitere Möglichkeit bietet die elektrolytische Entfettung. Bei dieser wird dem Elektrolyt ein alkalischer Reiniger zugesetzt und die Elektroden wechselnd polarisiert. Durch die Gasentwicklung an der Elektrodenoberfläche wird die reinigende Wirkung der alkalischen Lösung verstärkt. Allerdings wird dieses Verfahren bisher nur sehr eingeschränkt eingesetzt, weil zu befürchten ist, dass die Polarisierungsfähigkeit der Elektroden während der Messung durch die Zugabe der Base negativ beeinflusst wird.

Eine weitere Möglichkeit zur Reinigung wird in den Patentschriften CH 672845 A5 und EP 1 452 858 B1 offenbart. In diesen wird ein Verfahren beschrieben, bei dem in einem zum Messbetrieb separaten Reinigungsabschnitt eine Spannung wechselnder Polarität an die Arbeits- und Gegenelektrode angelegt wird, sodass abwechselnd sowohl reduzierende als auch oxidierende Gase an der Arbeits- und der Gegenelektrode gebildet werden. Hierdurch werden Verunreinigungen auf den Elektroden abgelöst und die aktiven Elektrodenoberflächen freigelegt. Aufgrund der im Reinigungsvorgang freiwerdenden Gase ist die Anwendung dieses Verfahrens für membranbedeckte Sensoren jedoch ausgeschlossen.

Hinzu kommt, dass bei allen oben genannten Verfahren im Anschluss an den Reinigungsvorgang die aktive Elektrodenoberfläche zuerst konditioniert werden muss, d.h. dass sich durch Gleichgewichtseinstellung eine messfähige Oberfläche ausbilden muss, sodass sich ein nahezu konstanter Blindwert und eine gleichbleibende Sensitivität des gemessenen Parameters, die für eine zuverlässige Messung notwendig sind, eingestellt hat. Das Konditionierungsverhalten ist abhängig von der Konzentration des zu bestimmenden Inhaltsstoffs in der Probe. Bei einer sehr geringen Konzentration des Inhaltsstoffs kann die Konditionierungsphase mehrere Stunden bis Tage andauern.

Ein weiteres Problem, das mit den Verfahren aus dem Stand der Technik gar nicht oder nur unzureichend gelöst werden kann, ergibt sich, wenn die zu messende Konzentration des Inhaltsstoffs unterhalb einer für den Sensortyp charakteristischen Schwelle liegt und damit nur ein sehr geringer oder zeitweise gar kein Stromfluss zwischen Arbeitselektrode und entsprechender Referenzelektrode auftritt ("Nullsensor"). Hierdurch kann es im Laufe der Zeit zur Veränderung der aktiven Elektrodenoberfläche kommen. Auch in diesem Fall muss die Arbeitselektrode neu konditioniert werden, bis sich die aktive Elektrodenoberfläche wieder ausgebildet hat und aussagekräftige Messwerte erhalten werden können.

Bei membranbedeckten amperometrischen Sensoren, wird die Messkammer durch eine für den zu bestimmenden Inhaltsstoff selektiv durchlässige Membran teilweise begrenzt. Diese Membran steht in unmittelbarem Kontakt mit der zu messenden Probe. Wenn aufgrund der Abwesenheit des Inhaltsstoffs der Sensor über längere Zeit inaktiv ist, können sich Ablagerungen, insbesondere organische Ablagerungen wie Biofilme, auf der Membranoberfläche oder innerhalb der Poren der Membran bilden. Dies führt zur Beeinträchtigung der Diffusion des Analyten durch die Membran hin zur Arbeitselektrode und kann dann die Sensorempfindlichkeit sowie die Messgenauigkeit beeinflussen. Als kostenerzeugender Wartungsschritt muss regelmäßig die Membrankappe, die die Membran einschließt, getauscht werden.

Die kommerziell erhältliche Messeinrichtung W&T/Siemens Deox/2000® löst einen Teil der oben genannten Probleme dadurch, dass der Messkammer kontinuierlich ein Konditioniermittel von außen zugeführt wird, das dann an der Arbeitselektrode umgesetzt wird. Dadurch wird die Elektrode dauerhaft in einem messfähigen Zustand gehalten. Das Konditioniermittel $I_2$ wird in einem der Messkammer vorgeschaltetem Reaktionsrohr durch die Redoxreaktion von KI mit Chloramin T hergestellt. Die wässrige $I_2$-Lösung wird anschließend mittels einer peristaltischen Pumpe in die Messkammer eingebracht. Für die Bestimmung der Konzentration eines Inhaltsstoffes einer Probe wird der $I_2$-Lösung vor Zuführung in die Messkammer eine definierte Menge an Probe beigemischt. Durch Reaktion des Iods mit dem oxidierenden oder reduzierenden Inhaltsstoff der Probe wird die Konzentration von Iod erhöht oder erniedrigt. Hierdurch verändert sich auch der Messwert des amperometrischen Sensors, wodurch auf die Konzentration des zu bestimmenden Inhaltsstoffes geschlossen werden kann. Allerdings erfordert dieses Verfahren nicht nur die Integration eines Reaktionsrohres in die Messeinrichtung, es müssen für den kontinuierlichen Betrieb entsprechende Mengen von reaktiven und teilweise instabilen Chemikalien wie Chloramin T vorgehalten werden. Diese können sich im Laufe der Aufbewahrungszeit zu ungewünschten Nebenprodukten zersetzen, die den Sensor verunreinigen. Weiterhin kann das Konditioniermittel nur auf umständliche Weise, nämlich durch Austausch der vorgehaltenen Chemikalien, gewechselt werden.

**Aufgabe**

Aufgabe der vorliegenden Erfindung ist es, ein Verfahren zur Reinigung, Konditionierung, Kalibration und/oder Justage eines membranbedeckten amperometrischen Sensors einer Messeinrichtung zur Bestimmung von Inhaltsstoffen einer Probe bereitzustellen, das eine vergleichbare oder sogar eine bessere Reinigungs- und Konditionierwirkung als die aus dem Stand der Technik bekannten Verfahren aufweist, für das ein einfacherer apparativer Aufbau ausreichend ist, insbesondere keine Chemikalien vorgehalten werden müssen, und das eine Verlängerung der Standzeit der Membran ermöglicht.

**Beschreibung der Erfindung**

Erfindungsgemäß wird diese Aufgabe gelöst durch ein Verfahren der oben genannten Art, das **dadurch gekennzeichnet ist, dass** es folgende Schritte umfasst:

> Erzeugen eines Konditioniermittels an der Arbeitselektrode und/oder an einer in der Messeinrichtung angeordneten Generatorelektrode, wobei das Konditioniermittel ein Oxidations- oder Reduktionsmittel ist,

> Oxidation des Konditioniermittels an der Arbeitselektrode, wenn das Konditioniermittel ein Reduktionsmittel ist oder Reduktion des Konditioniermittels an der Arbeitselektrode, wenn das Konditioniermittel ein Oxidationsmittel ist.

Durch die elektrochemische Umsetzung des Konditionierungsmittels wird die Arbeitselektrode gereinigt und in einen messfähigen Zustand mit einer aktiven Elektrodenoberfläche versetzt, sodass sich ein nahezu konstanter Blindwert und eine gleichbleibende Sensitivität des gemessenen Parameters, die für eine zuverlässige Messung notwendig sind, einstellt. Wenn sich die Arbeitselektrode bereits in einem messfähigen Zustand befindet, wird diese durch die elektrochemische Umsetzung des Konditioniermittels in diesem Zustand gehalten.

Ohne an die Theorie gebunden zu sein, gehen die Erfinder davon aus, dass die Konditionierung der Arbeitselektrode durch eine Entfernung von Ablagerungen und einer Depolarisierung erfolgt.

Erfindungsgemäß kann das Konditioniermittel an einer in der Messeinrichtung, vorzugsweise in der Messkammer, angeordneten Generatorelektrode und/oder an der in der Messkammer angeordneten Arbeitselektrode erzeugt werden. Wenn das Konditioniermittel an einer in der Messeinrichtung angeordneten Generatorelektrode aus einem im Elektrolyt enthaltenden Reduktions- oder Oxidationsmittel erzeugt wird, erreicht das Konditioniermittel durch Diffusion und/oder Transport durch eine Pumpe die in der Messkammer angeordnete Arbeitselektrode und kann an dieser durch Anlegen einer zur Umsetzung notwendigen Spannung umgesetzt werden. Hierdurch wird der Konditionierungseffekt erzielt. Die Erzeugung des Konditioniermittels kann während eines Messintervalls oder in einem separaten Konditionierintervall erfolgen. Vorzugsweise wird das Konditioniermittel wieder zu dem im Elektrolyt enthaltenden Oxidations- oder Reduktionsmittel umgesetzt. Alternativ oder zusätzlich kann das Konditioniermittel auch an der Arbeitselektrode erzeugt werden. Die Erzeugung des Konditioniermittels kann dann nur in einem separaten Konditionierintervall erfolgen, d.h. die Messung des Inhaltsstoffes muss pausiert und durch Anlegen einer entsprechenden Spannung zwischen Arbeits- und Arbeitsreferenzelektrode aus einem im Elektrolyt enthaltenden Reduktions- oder Oxidationsmittel das Konditioniermittel erzeugt werden. Im Anschluss kann durch Spannungsänderung die elektrochemische Redoxreaktion umgekehrt werden, d.h. das Konditioniermittel wird an der Arbeitselektrode nicht mehr erzeugt, sondern umgesetzt. Hierdurch tritt der Konditionierungseffekt ein. Vorzugsweise wird das Konditioniermittel wieder zu dem im Elektrolyt enthaltenen Oxidations- oder Reduktionsmittel umgesetzt.

Durch das erfindungsgemäße Verfahren können auch die weiteren Bestandteile des amperometrischen Sensors, insbesondere die Membran und die weiteren Elektroden, von Verunreinigungen, die die Diffusion und die elektrochemische Reaktion an der Arbeitselektrode beeinträchtigen, befreitwerden bzw. die Ablagerung von Verunreinigungen verzögert werden. Dies ist auf die oxidative oder reduktive Wirkung des Konditioniermittels, das sich im Elektrolyt durch Diffusion verbreiten kann, zurückzuführen.

Die "Messeinrichtung" ist das räumlich begrenzte Behältnis das mindestens einen amperometrischen Sensor und eine Steuervorrichtung umfasst. Sie kann weiterhin ein oder mehrere räumlich begrenzte Module zur Durchflussregelung, zur Messung des pH-Wertes und ein Modul mit mindestens einer Generatorelektrode sowie ein oder mehrere Pumpen aufweisen. Der amperometrische Sensor kann mit den gegebenenfalls vorhandenen weiteren Modulen über eine oder mehrere Verbindungen, die Elektrolyt enthalten, verbunden sein. Die Inhaltsstoffe der Probe und/oder das Konditioniermittel können über Pumpen zur Messkammer transportiert werden. Gleichzeitig oder alternativ können die Inhaltsstoffe und/oder das Konditioniermittel durch Diffusion zur Messkammer gelangen. Sie diffundieren dann durch die Membran und werden innerhalb der Messkammer an der Arbeitselektrode umgesetzt.

Der amperometrische Sensor umfasst eine Messkammer, die Elektrolyt enthält und in der die Arbeitselektrode und eine mit der Arbeitselektrode elektrisch verbundene Arbeitsreferenzelektrode angeordnet sind. Eine "Arbeitselektrode" ist im erfindungsgemäßen Zusammenhang diejenige Elektrode, die zur Bestimmung des Messwertes des amperometrischen Sensors dient. An dieser wird der zu bestimmende Inhaltsstoff der Probe elektrochemisch oxidiert oder reduziert. Hierzu wird während des Messvorgangs eine Spannung zwischen Arbeitselektrode und der mit dieser elektrisch verbundenen Referenzelektrode angelegt, wobei mit einer geeigneten Anordnung, wie beispielsweise einem Potentiostat, die Prozesse geregelt werden.

Die Bestimmung des Inhaltsstoffes während eines Messintervalls erfolgt dadurch, dass der Strom, der über die elektrische Verbindung von Arbeitselektrode und Arbeitsreferenzelektrode fließt, gemessen wird, und aus dem gemessenen Strom auf den Inhaltsstoff rückgeschlossen wird.

In einer Ausführungsform umfasst der amperometrische Sensor weiterhin eine Arbeitsgegenelektrode, die ebenfalls elektrisch mit der Arbeitsreferenzelektrode verbunden ist, und an der eine Redoxreaktion zum Ladungsausgleich abläuft. Eine entsprechende Ausführungsform des Arbeitselektrodensystems wird als Drei-Elektroden-Anordnung bezeichnet. In einer bevorzugten Ausführungsform kann die Arbeitsreferenzelektrode gleichzeitig auch die Arbeitsgegenelektrode sein. Diese Ausführung wird als Zwei-Elektroden-Anordnung bezeichnet.

Der Begriff "Elektrolyt" umfasst im Rahmen der vorliegenden Erfindung ionenleitende Medien, insbesondere ionenleitende Flüssigkeiten, wie beispielsweise Salzlösungen oder salzhaltige Gele.

Der Messwert des amperometrischen Sensors ist die Stromstärke, die zwischen der Arbeitselektrode und der Arbeitsreferenzelektrode gemessen wird.

Die "Probe", deren Inhaltsstoff bestimmt werden soll, ist eine Flüssigkeit, vorzugsweise eine ionenleitende, wie beispielsweise Wasser.

In einer Ausführungsform besteht die Arbeitselektrode aus einem Edelmetall, bevorzugt aus Platin oder Gold, besonders bevorzugt aus Platin. In einer anderen Ausführungsform besteht die Arbeitselektrode aus Glaskohlenstoff bzw. anderen in der Literatur bekannten Elektrodenmaterialien.

Das "Konditioniermittel", das in der Messeinrichtung erzeugt wird und an der Arbeitselektrode des amperometrischen Sensors reduziert wird, kann ein Oxidationsmittel sein. Oxidationsmittel bedeutet in diesem Zusammenhang, dass das Redoxpaar aus Oxidationsmittel und dem korrespondierenden Reduktionsmittel ein Redoxpotential aufweist, sodass das Oxidationsmittel durch die an der Arbeitselektrode angelegte Spannung zum korrespondieren Reduktionsmittel reduziert wird. D.h. dass die Spannung zwischen Arbeits- und elektrisch verbundener Arbeitsreferenzelektrode kleiner als das Standardredoxpotential des Redoxpaares bestehend aus Oxidationsmittel und dem korrespondierenden Reduktionsmittel ist.

Ist das Konditioniermittel beispielsweise $I_2$, so muss unter Standardbedingungen die Arbeitselektrode gegenüber der Arbeitsreferenzelektrode <540 mV polarisiert sein, da das Standardredoxpotential des Redoxpaares $I_2/2\ I^-$ 540 mV beträgt. Ist die Spannung unterhalb dieses Grenzwertes, wird Iod zu Iodid reduziert:

$$I_2 + 2\ e^- \rightarrow 2\ I^-$$

Wird nicht unter Standardbedingungen gearbeitet, so muss die anzuwendende Spannung, wie es dem Fachmann bekannt ist, in entsprechender Weise angepasst werden. Unter anderem sind die bekannten Überspannungen für verschiedene Elektrodenmaterialien zu berücksichtigen. Das Konditioniermittel, das in der Messeinrichtung erzeugt wird, kann auch ein Reduktionsmittel sein. Reduktionsmittel bedeutet in diesem Zusammenhang, dass das Redoxpaar aus Reduktionsmittel und dem korrespondierenden Oxidationsmittel ein Redoxpotential aufweist, sodass das Reduktionsmittel durch die an der Arbeitselektrode angelegte Spannung zum korrespondierenden Oxidationsmittel oxidiert wird.

In einer bevorzugten Ausführungsform der Erfindung wird das Konditioniermittel an der Generatorelektrode erzeugt. Eine "Generatorelektrode" ist im erfindungsgemäßen Zusammenhang eine Elektrode, die zur elektrolytischen Erzeugung eines oder mehrerer Konditioniermittel in der Messeinrichtung dient. Dazu wird eine Spannung zwischen Generatorelektrode und einer mit dieser verbundenen Generatorreferenzelektrode in der Höhe angelegt, so, dass ein Konditioniermittel aus einem Reduktions- oder Oxidationsmittel, das im Elektrolyt enthalten ist, erzeugt wird. Hierzu kann das Potential zwischen der Generatorelektrode und der Generatorreferenzelektrode mit einer geeigneten Anordnung, wie beispielsweise einem Potentiostat geregelt werden. In einer Ausführungsform umfasst die Messeinrichtung weiterhin eine Generatorgegenelektrode, die ebenfalls elektrisch mit der Generatorreferenzelektrode verbunden ist, und an der eine Redoxreaktion zum Ladungsausgleich abläuft. Eine entsprechende Ausführungsform des Generatorelektrodensystems wird als Drei-Elektroden-Anordnung bezeichnet. In einer bevorzugten Ausführungsform kann die Generatorreferenzelektrode gleichzeitig auch die Generatorgegenelektrode sein. Diese Ausführung wird als Zwei-Elektroden-Anordnung bezeichnet. Die in der Messkammer angeordnete Arbeitselektrode und die in der Messeinrichtung angeordnete Generatorelektrode können ein und dieselbe Referenzelektrode aufweisen. Weiterhin bevorzugt haben Arbeits- und Generatorelektrode ein und dieselbe Gegenelektrode. Durch gemeinsame Nutzung von Elektroden kann die Anzahl der Elektroden in der Messeinrichtung entsprechend reduziert werden. Beispielsweise kann die Gesamtelektrodenanzahl, die bei einer Drei-Elektronen-Anordnung des Arbeitselektrodensystems und einer Drei-Elektroden-Anordnung des Generatorelektrodensystems 6 beträgt, durch gemeinsame Nutzung der gleichen Referenz- und Gegenelektroden auf 4 reduziert werden.

In einer Ausführungsform besteht die Generatorelektrode aus einem Edelmetall, bevorzugt aus Platin oder Gold, besonders bevorzugt aus Platin. In einer anderen Ausführungsform besteht die Arbeitselektrode aus Glaskohlenstoff.

In einer weiteren bevorzugten Ausführungsform besteht die Generatorelektrode aus Titan.

Die Generatorelektrode kann beispielsweise $I_2$ als Konditioniermittel erzeugen. Hierzu muss der Elektrolyt das korrespondierende Reduktionsmittel, Iodid, enthalten und die Generatorelektrode gegenüber der Generatorreferenzelektrode >540 mV polarisiert sein, da das Standardredoxpotential des Redoxpaares $I_2$/2 $I^-$ 540 mV beträgt. Ist dieser Grenzwert überschritten, wird Iodid zu Iod oxidiert:

$$2I^- \rightarrow I_2 + 2\ e^-\ \text{Generatorelektrodenreaktion}$$

Das entstandene Konditioniermittel Iod diffundiert dann zur Arbeitselektrode. Ist diese gegenüber der Arbeitsreferenzelektrode <540 mV polarisiert, so wird dort Iod wieder zu Iodid reduziert, wodurch die konditionierende Wirkung eintritt.

$$I_2 + 2\ e^- \rightarrow 2\ I^-\ \text{Arbeitselektrodenreaktion}$$

Damit ein Konditioniermittel an der Generator- oder Arbeitselektrode erzeugt werden kann, muss der Elektrolyt das zum Konditioniermittel korrespondierende Reduktions- oder Oxidationsmittel, aus dem das Konditioniermittel erzeugt wird, in ausreichender Konzentration aufweisen. Dies kann beispielsweise dadurch erreicht werden, dass der in der Messeinrichtung enthaltende Elektrolyt eine Salzlösung, beispielsweise eines Metalliodidsalzes, ist.

Das Konditioniermittel kann auch dadurch erzeugt werden, dass an der Generatorelektrode ein Reduktions- oder Oxidationsmittel erzeugt wird, das ein im Elektrolyt enthaltendes Oxidations-/Reduktionsmittel zum Konditioniermittel reduzieren oder oxidieren kann.

Soll das Konditioniermittel beispielsweise $I_2$ sein, so kann dies in der Messeinrichtung auch dadurch erzeugt werden, dass der Elektrolyt Iodidionen enthält und ein Oxidationsmittel in der Messeinrichtung an der Generatorelektrode erzeugt, das Iodid zu $I_2$ oxidieren kann.

Ist das Oxidationsmittel beispielsweise $Br_2$, so wird dieses unter Standardbedingungen von $I^-$ reduziert und es entsteht das Konditioniermittel $I_2$:

$$\begin{array}{ll} Br_2 + 2e^- \rightarrow 2\ Br^- & \text{Reduktion} \\ 2\ I^- \rightarrow I_2 + 2\ e^- & \text{Oxidation} \\ \hline Br_2 + 2\ I^- \rightarrow 2\ Br^- + I_2 & \text{Gesamt} \end{array}$$

Durch die Reaktion entsteht $I_2$, das, wie oben dargestellt, durch Anlegen entsprechender Spannung an der Arbeitselektrode reduziert wird. Durch die Reaktion an der Arbeitselektrode wird der Konditioniereffekt erzielt.

Bevorzugt ist das Konditioniermittel ein Oxidationsmittel, besonders bevorzugt Brom, Chlor, Iod, am bevorzugtesten Iod.

Das durch die Arbeitselektrode oder durch die Generatorelektrode erzeugte Konditioniermittel bzw. das korrespondierende Oxidations-/Reduktionsmittel kann durch die Poren bzw. durch das Material der Membran hindurchtreten. Die Anlagerung von Stoffen, insbesondere organischen Stoffen wie Biofilmen, auf der Oberfläche oder innerhalb der Poren der Membran kann durch Oxidation oder Reduktion der Stoffe verzögert bzw. verringert werden. Damit kann die Standzeit der Membran deutlich verlängert werden.

In einer Ausführungsform der Erfindung wird das Konditioniermittel an der Generatorelektrode während des Messintervalls erzeugt. In diesem Fall ist der Referenzwert, der durch das zugeführte oder erzeugte Konditioniermittel an der Arbeitselektrode hervorgerufen wird, vom Messwert des amperometrischen Sensors zu subtrahieren.

Hierdurch kann ein kontinuierlicher Messbetrieb gewährleistet werden, d.h. die Elektrodenoberfläche der Arbeitselektrode bleibt aktiv und es sind keinerlei Reinigungs- und/oder Konditionierungsphasen notwendig.

In einer weiteren Ausführungsform der Erfindung wird das Konditioniermittel in einem separaten Reinigungs- und/oder Konditionierungsvorgang an der Arbeitselektrode oder an der Generatorelektrode erzeugt. Es ist vorteilhaft, dass das Konditionierungsverhalten der Elektroden von der Konzentration des Konditioniermittels abhängig ist. Durch die Erzeugung einer entsprechend hohen Konzentration an Konditioniermittel kann die Konditionierungsphase verkürzt werden.

In einer weiteren Ausführungsform wird das Konditioniermittel diskontinuierlich, bevorzugt pulsförmig, erzeugt, wobei das zeitliche Intervall, in dem das Konditioniermittel erzeugt wird, sehr viel kürzer ist, als das zeitliche Intervall, in dem kein Konditioniermittel erzeugt wird. Wird das Konditioniermittel an der Generatorelektrode während des Messbetriebs erzeugt, so ist der Referenzwert, der durch das Konditioniermittel an der Arbeitselektrode hervorgerufen wird, vom Messwert des Sensors zu subtrahieren. Alternativ kann die Messung für das kurze zeitliche Intervall, in dem das Konditioniermittel erzeugt wird, auch pausiert werden.

In einer Ausführungsform der Erfindung wird das Konditioniermittel kontinuierlich erzeugt. Wenn die kontinuierliche Erzeugung an der Generatorelektrode während des Messintervalls erfolgt, wird ein definierter, nahezu konstanter Referenzwert durch das Konditioniermittel an der Arbeitselektrode erhalten, der vom Messwert der Arbeitselektrode zu subtrahieren ist. Durch die kontinuierliche Erzeugung des Konditioniermittels wird dann eine gleichmäßige Reinigungs- und Konditionierungswirkung erzielt. Dadurch wird verhindert, dass sich Verunreinigungen auf der Oberfläche der Arbeitselektrode festsetzen können und die Struktur der Elektrodenoberfläche der Arbeitselektrode sich in der Art verändert, dass sie inaktiv wird. Folglich kann die Gesamtmenge an Konditioniermittel, das an der Generatorelektrode erzeugt wird, gering gehalten werden.

In einer bevorzugten Ausführungsform wird eine vorbestimmte Menge an Konditioniermittel in der Messeinrichtung erzeugt.

In einer Ausführungsform der Erfindung handelt es sich beim Konditioniermittel, das erzeugt wird, um den Inhaltsstoff der Probe, der von dem amperometrischen Sensor bestimmt wird. Dies kann vorteilhaft dazu genutzt werden, den Inhaltsstoff einer Probe zu bestimmen, dessen Konzentration unterhalb der Nachweisgrenze eines Sensors ist, bei dem kein Konditioniermittel in die Messkammer zugeführt oder in dieser erzeugt wird:
Die Nachweisgrenze eines Sensors bezeichnet den Wert eines Messverfahrens, bis zu dem die Messgröße gerade noch zuverlässig nachgewiesen werden kann.

Der Messwert an der Nachweisgrenze hat eine erhöhte Ungenauigkeit, die aber ein vorgegebenes statistisches Konfidenzintervall nicht überschreitet. Messwerte, die eine größere Ungenauigkeit aufweisen als das vorgegebene Intervall, liegen unterhalb der Nachweisgrenze und werden im Sinne der Messtechnik als unmessbar bzw. nicht nachweisbar bezeichnet.

Das Kriterium des "zuverlässigen Nachweises" wird in der Regel bezogen auf die Präzision des Messverfahrens bei einer den Blindwert ergebenden Leermessung. Gemeint ist damit der statistische Fehler oder die Schwankung des Messsignals, wenn keine Probe vorhanden ist (z.B. die Standardabweichung vom Blindwert).

Im erfindungsgemäßen Zusammenhang gilt eine Messung als Nachweis, wenn der Messwert mindestens drei Standardabweichungen über dem Blindwert liegt.

Um den Inhaltsstoff einer Probe zu bestimmen, dessen Konzentration unterhalb der Nachweisgrenze einer Messeinrichtung liegt, bei der kein Konditioniermittel in der Messeinrichtung erzeugt wird, wird der zu bestimmende Inhaltsstoff während der Messung in der Messeinrichtung an der Generatorelektrode oder vor der Messung an der Arbeitselektrode erzeugt. Bei der Umsetzung an der Arbeitselektrode wird ein Messwert erhalten, der sich als Summe des Referenzwertes, der durch das Konditioniermittel an der Arbeitselektrode erzeugt wird, und dem Wert, der durch den Inhaltsstoff der Probe an der Arbeitselektrode erzeugt wird, ergibt. Der erhaltene Messwert kann so auf einen Wert oberhalb der Nachweisgrenze angehoben werden. Durch Subtraktion des Referenzwertes vom Messwert kann der Inhaltsstoff im Elektrolyten bestimmt werden.

In einer weiteren Ausführungsform der Erfindung handelt es sich beim Konditioniermittel, das an der Arbeits- oder Generatorelektrode erzeugt wird, nicht um den Inhaltsstoff, der von dem amperometrischen Sensor bestimmt wird. In einer weiteren Ausführungsform können auch mehrere unterschiedliche Oxidations- und/oder Reduktionsmittel an der Generator- oder Arbeitselektrode erzeugt werden. Vorteilhaft ist beim erfindungsgemäßen Verfahren, dass das Konditioniermittel sehr einfach gewechselt werden kann. Wenn der Elektrolyt bereits verschiedene korrespondierende Oxidations- bzw. Reduktionsmittel enthält, kann ein Wechsel bereits durch die Anpassung der Spannung an der Arbeits- oder Generatorelektrode erfolgen. Der Wechsel des Konditioniermittels kann auch dadurch erfolgen, dass dem Elektrolyt ein nicht bereits enthaltendes korrespondierendes Oxidations- bzw. Reduktionsmittel beigefügt wird und die Spannung an der Arbeits- oder Generatorelektrode so eingestellt wird, dass dieses zum Konditioniermittel umgesetzt wird.

In einer Ausführungsform wird als Elektrolyt ein Detektionselektrolyt verwendet. Ein Detektionselektrolyt weist einen Bestandteil auf, der durch den zu bestimmenden Inhaltsstoff der Probe zu einem Detektionsbestandteil reduziert oder oxidiert wird. Der Detektionsbestandteil wird an der Arbeitselektrode oxidiert oder reduziert und es wird anhand der erhaltenen Stromstärke auf den zu bestimmenden Inhaltsstoff geschlossen. Wird beispielsweise als Detektionselektrolyt einer Messeinrichtung zur Bestimmung von $Cl_2$ in einer Probe eine Iodidsalzlösung eingesetzt, so oxidiert der zu bestimmende Inhaltsstoff der Probe, $Cl_2$, das im Detektionselektrolyt enthaltende $I^-$ zu dem Detektionsbestandteil $I_2$.

$$Cl_2 + 2e^- \rightarrow 2\,Cl^- \qquad \text{Reduktion}$$
$$2\,I^- \rightarrow I_2 + 2\,e^- \qquad \text{Oxidation}$$
$$\overline{Cl_2 + 2\,I^- \rightarrow 2\,Cl^- + I_2} \qquad \text{Gesamt}$$

Der Detektionsbestandteil $I_2$ kann dann an der Arbeitselektrode reduziert werden und über die als Messwert erhaltene Stromstärke auf den Inhaltsstoff $Cl_2$ geschlossen werden.

In einer bevorzugten Ausführungsform wird als Konditioniermittel der Detektionsbestandteil verwendet.

In einer bevorzugten Ausführungsform ist der zu bestimmende Inhaltsstoff der Probe ein Oxidationsmittel, das an der Arbeitselektrode reduziert wird, wie beispielsweise oxidativ wirkende Halogenverbindungen wie die des Chlor, Broms und Iods, Chlor- und Bromamine, $Cl_2$, $Br_2$, $O_3$, $ClO_2$, Peressigsäure, $H_2O_2$, ein Chlorit- oder Hypochloritsalz bzw. die korrespondierende Säure, vorzugsweise Hypochlorige Säure (HOCl).

In einer bevorzugten Ausführungsform ist der Elektrolyt eine Iodidsalzlösung und das Konditioniermittel $I_2$.

In einer bevorzugten Ausführungsform wird das erfindungsgemäße Verfahren zur Überwachung der Funktionsfähigkeit des Sensors genutzt. Hierzu wird an der Generatorelektrode eine definierte Menge des zu bestimmenden Inhaltsstoffs erzeugt und eine Spannung zwischen Arbeitselektrode und Arbeitsreferenzelektrode angelegt und der Strom, der über die elektrische Verbindung von Arbeitselektrode und Arbeitsreferenzelektrode fließt, gemessen und mit einem vorbekannten Wert verglichen werden. So können etwaige Fehler, wie ein Sensordefekt, Kabelunterbrechungen oder Fehler im Auswertesystem erkannt werden.

In einer bevorzugten Ausführungsform wird von dem während des Messintervalls gemessenen Strom ein Justagewert abgezogen, wobei der Justagewert dadurch bestimmt wird, das während eines Justageintervalls, bei dem kein zu bestimmender Inhaltsstoff von einer Probe im Elektrolyt vorhanden ist, an der Generatorelektrode eine definierte Menge des zu bestimmenden Inhaltsstoffes erzeugt wird und eine Spannung zwischen Arbeitselektrode und Arbeitsreferenzelektrode angelegt wird und der Strom, der über die elektrische Verbindung von Arbeitselektrode und Arbeitsreferenzelektrode fließt, gemessen wird.

In einer weiteren bevorzugten Ausführungsform wird das Konditioniermittel innerhalb der Messkammer durch die Generatorelektrode erzeugt. Besonders vorteilhaft ist es, die Generatorelektrode als Anode zu betreiben, wenn die Arbeitselektrode als Kathode verwendet wird, und die Generatorelektrode als Kathode zu betreiben, wenn die Arbeitselektrode als Anode verwendet wird.

Die Erfindung umfasst auch einen amperometrischen Sensor zur Durchführung des oben definierten Verfahrens, wobei der Sensor mindestens eine in einer den Elektrolyten enthaltenden Messkammer angeordnete Arbeitselektrode, eine Arbeitsreferenzelektrode und eine selektiv durchlässige Membran, welche die Messkammer begrenzt, umfasst, wobei in der Messkammer eine Generatorelektrode und eine Generatorreferenzelektrode angeordnet sind und wobei eine Steuervorrichtung vorgesehen ist, mit der zwischen Arbeitselektrode und Arbeitsreferenzelektrode eine Messspannung angelegt werden kann und mit der zwischen Generatorelektrode und Generatorreferenzelektrode eine Generatorspannung angelegt werden kann.

Beim erfindungsgemäßen Verfahren ergibt sich die Diffusionsrichtung des Konditioniermittels aus dessen Konzentrationsgradienten. In einer Ausführungsform der Erfindung wird das Konditioniermittel an einer in der Messeinrichtung angeordneten Generatorelektrode erzeugt. Die Konzentration des Konditioniermittels ist an der Generatorelektrode hoch, da es dort aus dem korrespondierenden Oxidations-/Reduktionsmittel erzeugt wird. An der Arbeitselektrode wird das Konditioniermittel hingegen umgesetzt, sodass die Konzentration dort normalerweise geringer ist. Das Konditioniermittel strömt daher im Regelfall bei einer Anordnung der Generatorelektrode außerhalb der Messkammer in diese hinein und wird dort umgesetzt. Wenn die Generatorelektrode jedoch innerhalb der Messkammer angeordnet ist, ist die Konzentration außerhalb der Messkammer in der Regel am geringsten. Das an der Generatorelektrode erzeugte Konditioniermittel strömt Großteils durch die durchlässige Membran aus der Messkammer hinaus und kann daher nicht konditionierend wirken. Wenn die Generatorelektrode innerhalb der Messkammer angeordnet ist, ist es daher vorteilhaft, die Arbeitselektrode in der Diffusionsrichtung des Konditioniermittels anzuordnen, um die reinigende und konditionierende Wirkung des Konditioniermittels zu maximieren. Wird die Arbeitselektrode nun in dieser Richtung angeordnet, wird das Konditioniermittel dort vor Austritt aus der Membran zum korrespondierenden Reduktions- oder Oxidationsmittel oxidiert oder reduziert, welches dann zurück zur Generatorelektrode diffundiert. Hierdurch bildet sich ein geschlossener Kreislauf, wodurch nahezu kein Oxidations- oder Reduktionsmittel aus der Membran herausdiffundieren kann.

Um eine solche Anordnung von Arbeitselektrode und Generatorelektrode zu erreichen, kann in einer Ausführungsform die Arbeitselektrode zumindest einen zylinderförmigen Abschnitt aufweisen und die Generatorelektrode einen hohlzylinderförmigen Abschnitt, wobei die Arbeitselektrode mit ihrem zylinderförmigen Abschnitt innerhalb des hohlzylinderförmigen Abschnitts der Generatorelektrode angeordnet ist. Vorzugsweise ist die Arbeitselektrode innerhalb des hohlzylinderförmigen Abschnitts der Generatorelektrode in der Art angeordnet, dass beide zur selektiv durchlässigen Membran weisenden Grundflächen der Arbeits- und Generatorelektroden bündig miteinander abschließen oder die Grundfläche der Generatorelektrode einen größeren Abstand zur selektiv durchlässigen Membran aufweist, als die Grundfläche der Arbeitselektrode. Hierdurch wird erreicht, dass das Konditioniermittel, das an der Generatorelektrode erzeugt wird, an der Arbeitselektrode oxidiert oder reduziert wird und in nur geringem Maße durch die Membran nach außen in die Probe diffundiert.

Vorzugsweise ist die effektive Fläche der selektiv durchlässigen Membran, d.h. die durchlässige Fläche, kleiner oder gleich der der Membran zugewandten Grundfläche der Arbeitselektrode. Hierdurch wird erreicht, dass die Strömungsrichtung des an der Generatorelektrode erzeugten Konditioniermittels eine radial nach innen, d.h. zur Arbeitselektrode gerichtete Komponente aufweist, das Konditioniermittel folglich zu dieser diffundiert, und dort reduziert oder oxidiert wird.

In einer besonders bevorzugten Variante dieser Ausführungsform ist die Arbeitselektrode in der Diffusionsrichtung des Konditioniermittels folgendermaßen angeordnet:

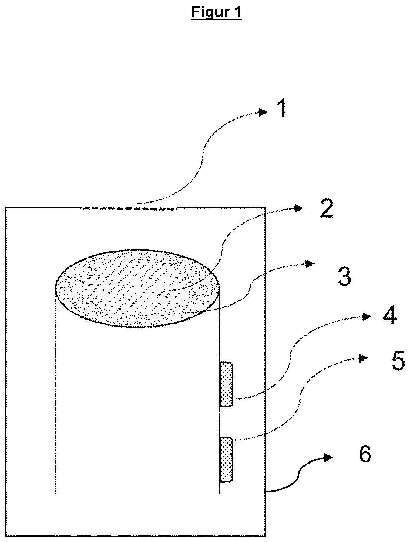

Die Arbeitselektrode ist zylindrisch und die Generatorelektrode hohlzylindrisch ausgebildet, wobei die Arbeitselektrode mit kleinerem Durchmesser innerhalb des Hohlzylinders der Generatorelektrode angeordnet ist. Auf der zur Membran ausgerichteten Seite schließen die Grundflächen der beiden Zylinder bündig miteinander ab. Der selektiv durchlässige Abschnitt der Membran ist kleiner als die Grundfläche des Zylinders der Arbeitselektrode und so angeordnet, dass dessen Projektion auf die Grundfläche der Arbeitselektrode zu mindestens 90% auf der Grundfläche der Arbeitselektrode liegt. Bevorzugt ist der selektiv durchlässige Abschnitt der Membran konzentrisch zur Grundfläche des Zylinders der Arbeitselektrode angeordnet. Figur 1 zeigt eine Vorrichtung, die für die Ausführung eines solchen bevorzugten Verfahrens geeignet ist.

Die Erfindung umfasst auch eine Messeinrichtung zur Durchführung des oben definierten Verfahrens, wobei die Einrichtung mindestens einen amperometrischen Sensor und eine Steuervorrichtung aufweist, sowie mindestens eine in der Messeinrichtung angeordnete Generatorelektrode. Mit der Steuervorrichtung kann zwischen Arbeitselektrode und Arbeitsreferenzelektrode eine Messspannung und zwischen Generatorelektrode und Generatorreferenzelektrode eine Generatorspannung angelegt werden, deren Betrag unterschiedlich oder gleich zu dem der Messspannung sein kann, deren Polarität jedoch entgegengesetzt ist.

Die anhängenden Figuren repräsentieren spezielle Ausführungsformen der Erfindung, dabei zeigen:

Figur 1:  Schematische Darstellung einer speziellen Ausführungsform einer erfindungsgemäßen Messkammer mit selektiv durchlässiger Membran und darin angeordneter Generatorelektrode.

Figur 2:  Schematische Darstellung eines Ausschnitts einer speziellen Ausführungsform einer erfindungsgemäßen Messkammer mit selektiv durchlässiger Membran und darin angeordneten Generatorelektroden.

Figur 3:  Schematische Darstellung einer speziellen Ausführungsform einer erfindungsgemäßen Messeinrichtung mit einer Messkammer mit selektiv durchlässiger Membran und in räumlicher Nähe zur Messkammer angeordneten Generatorelektroden.

Figur 4:  Schematische Darstellung einer speziellen Ausführungsform einer erfindungsgemäßen Messeinrichtung mit einer Messkammer mit selektiv durchlässiger Membran und einem separatem Modul, das die Generatorelektrode aufweist.

Figur 1 zeigt eine schematische Darstellung einer Messkammer 6 eines erfindungsgemäßen amperometrischen Sensors, die eine selektiv durchlässige Membran 1, eine Arbeitselektrode 2, eine Generatorelektrode 3 und die dazugehörigen Referenz- bzw. Gegenelektroden 4 und 5 aufweist. Arbeitselektrode 2 und Generatorelektrode 3 sind zylindrisch mit unterschiedlichen Durchmessern ausgebildet, wobei die Arbeitselektrode mit kleinerem Durchmesser innerhalb der hohlzylindrischen Generatorelektrode ausgebildet ist. Die der selektiv durchlässigen Membran zugewandten Grundflächen der Arbeits- und Generatorelektroden schließen bündig miteinander ab, wobei die Projektion der effektiven Fläche der Membran lediglich auf der Grundfläche der Arbeitselektrode liegt und kleiner als die Grundfläche der Arbeitselektrode ist.

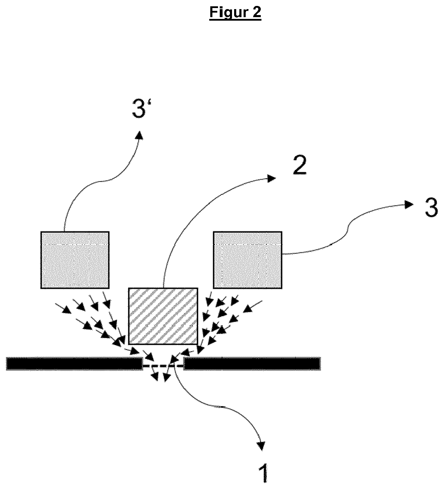

Figur 2 zeigt einen Ausschnitt aus der Darstellung einer speziellen Ausführungsform eines erfindungsgemäßen amperometrischen Sensors, mit einer selektiv durchlässigen Membran 1, einer Arbeitselektrode 2 und Generatorelektroden 3, 3'. Die Arbeitselektrode weist einen kürzeren Abstand zur effektiven Fläche der selektiv durchlässigen Membran als die Generatorelektroden auf und die effektive Fläche der Membran ist kleiner als die zur effektiven Fläche gerichtete Fläche der Arbeitselektrode. Weiterhin sind die beiden Flächen konzentrisch ausgebildet. Der Ausschnitt zeigt auch die Strömungsvektoren des an der Generatorelektrode gebildeten Konditioniermittels. Die Arbeitselektrode ist in der Diffusionsrichtung des Konditioniermittels angeordnet. Hierdurch wird die reinigende und konditionierende Wirkung des Oxidations- oder Reduktionsmittels erhöht.

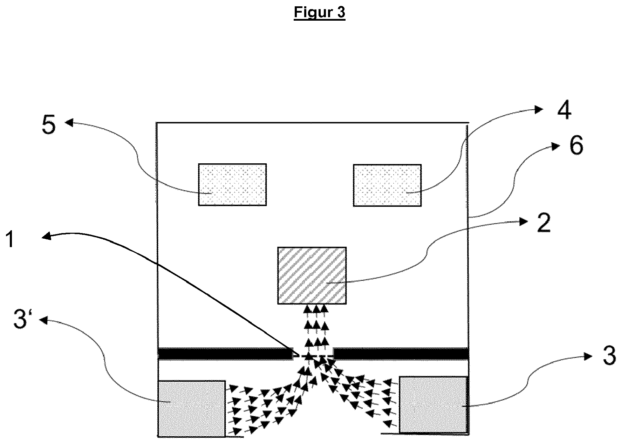

Figur 3 zeigt schematisch eine spezielle Ausführungsform einer erfindungsgemäßen Messeinrichtung mit einem amperometrischen Sensor und einer Anordnung der Generatorelektroden 3, 3' außerhalb der Messkammer 6. Dabei sind die das Konditioniermittel erzeugende Generatorelektroden 3, 3' in kurzem Abstand vor der Membran angeordnet. Das Konditioniermittel kann durch die selektiv durchlässige Membran 1 hindurch diffundieren und die Arbeitselektrode erreichen, an der es zum korrespondierenden Reduktions-/Oxidationsmittel reduziert wird. Die Strömungsvektoren des an der Generatorelektrode gebildeten Konditioniermittels sind ebenfalls dargestellt. Zur Vereinfachung wurden die Steuervorrichtung und die Generatorreferenzelektrode nicht in die Figur mitaufgenommen.

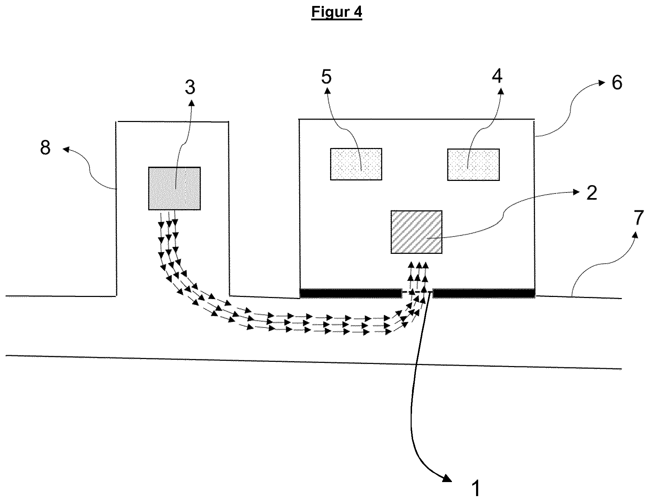

Figur 4 zeigt schematisch eine spezielle Ausführungsform einer erfindungsgemäßen Messeinrichtung mit amperometrischem Sensor und einem Modul 8, in der die Generatorelektrode 3 angeordnet ist. Das an der Generatorelektrode erzeugte Konditionierungsmittel diffundiert vom Modul 8 über die Verbindung 7 hin zur Messkammer 6, durchtritt deren selektiv durchlässige Membran 1 und wird an der Arbeitselektrode 2 reduziert. Die Strömungsvektoren des an der Generatorelektrode gebildeten Konditioniermittels sind ebenfalls dargestellt. Zur Vereinfachung wurden die Steuervorrichtung und die Generatorreferenzelektrode nicht in die Abbildung mitaufgenommen.

Bezugszeichenliste

1        Selektiv durchlässige Membran
2        Arbeitselektrode
3, 3'     Generatorelektrode
4, 5     Referenz- bzw. Gegenelektrode
6        Messkammer
7        Verbindung
8        Modul mit Generatorelektrode

**Patentansprüche**

1.  Verfahren zur Reinigung, Konditionierung, Kalibration und/oder Justage eines amperometrischen Sensors einer Messeinrichtung zur Bestimmung eines Inhaltsstoffes einer Probe, wobei die Messeinrichtung einen Elektrolyt und den amperometrischen Sensor mit einer selektiv durchlässigen Membran verschlossenen Messkammer, in der die Arbeitselektrode und eine mit der Arbeitselektrode elektrisch verbundene Arbeitsreferenzelektrode angeordnet sind, aufweist, und wobei die Bestimmung des Inhaltsstoffes während eines Messintervalls dadurch erfolgt, dass eine Spannung zwischen Arbeitselektrode und Arbeitsreferenzelektrode angelegt wird, der Strom, der über die elektrische Verbindung von Arbeitselektrode und Arbeitsreferenzelektrode fließt, gemessen wird, und aus dem gemessenen Strom auf den Inhaltsstoff rückgeschlossen wird, **dadurch gekennzeichnet, dass** das Verfahren folgende Schritte umfasst:

    Erzeugen eines Konditioniermittels an der Arbeitselektrode und/oder an einer in der Messeinrichtung angeordneten Generatorelektrode, wobei das Konditioniermittel ein Oxidations- oder Reduktionsmittel ist,
    Oxidation des Konditioniermittels an der Arbeitselektrode, wenn das Konditioniermittel ein Reduktionsmittel ist oder Reduktion des Konditioniermittels an der Arbeitselektrode, wenn das Konditioniermittel ein Oxidationsmittel ist.

2.  Verfahren nach Anspruch 1, **dadurch gekennzeichnet, dass** die Generatorelektrode, an der das Konditioniermittel erzeugt wird, in der Messkammer angeordnet ist.

**3.** Verfahren nach Anspruch 1 oder 2, **dadurch gekennzeichnet, dass** die Generatorelektrode das Konditioniermittel während des Messintervalls erzeugt, oder das Konditioniermittel während eines Konditionierintervalls erzeugt wird, wobei vorzugsweise das Konditioniermittel während mehrerer Konditionierintervalle zugeführt oder erzeugt wird, wobei die Konditionierintervalle kürzer sind als die Intervalle zwischen zwei aufeinanderfolgenden Konditionierintervallen.

**4.** Verfahren nach Anspruch 1 bis 4, **dadurch gekennzeichnet, dass** das Konditioniermittel kontinuierlich erzeugt wird, und/oder eine vorbestimmte Menge an Konditioniermittel erzeugt wird.

**5.** Verfahren nach einem der Ansprüche 1 bis 4, **dadurch gekennzeichnet, dass** als Konditioniermittel der zu bestimmende Inhaltsstoff verwendet wird.

**6.** Verfahren nach einem der vorhergehenden Ansprüche 1 bis 5, **dadurch gekennzeichnet, dass** als Elektrolyt ein Detektionselektrolyt verwendet wird, der einen Bestandteil aufweist, welcher durch den zu bestimmenden Inhaltsstoff in einen Detektionsbestandteil reduziert oder oxidiert wird, wobei vorzugsweise als Konditioniermittel der Detektionsbestandteil verwendet wird.

**7.** Verfahren nach einem der Ansprüche 1 bis 6, **dadurch gekennzeichnet, dass** der zu bestimmende Inhaltsstoff ein Oxidationsmittel ist, wie beispielsweise oxidativ wirkende Halogenverbindungen des Chlors, Broms und Iods, Chlor- und Bromamine, $Cl_2$, $Br_2$, $O_3$, $ClO_2$, Peressigsäure, $H_2O_2$, ein Chlorit- oder Hypochloritsalz bzw. die korrespondierende Säure, vorzugsweise Hypochlorige Säure (HOCl), und/oder als Elektrolyt eine Iodidlösung verwendet wird, wobei vorzugsweise als Konditioniermittel Iod verwendet wird.

**8.** Verfahren nach einem der Ansprüche 1 bis 7, **dadurch gekennzeichnet, dass** von dem während des Messintervalls gemessenen Strom ein Justagewert abgezogen wird, wobei der Nullwert dadurch bestimmt wird, dass während eines Justierintervalls, bei dem kein zu bestimmender Inhaltsstoff vorhanden ist, eine Spannung zwischen Arbeitselektrode und Arbeitsreferenzelektrode angelegt wird und der Strom, der über die elektrische Verbindung von Arbeitselektrode und Arbeitsreferenzelektrode fließt, gemessen wird.

**9.** Verfahren zur Kalibration und/oder Justage einer Messeinrichtung nach einem der Ansprüche 1 bis 8, **dadurch gekennzeichnet, dass** das Konditioniermittel dem zu bestimmenden Inhaltsstoff der Probe entspricht und eine definierte Menge Konditioniermittel durch Elektrolyse in der Messkammer erzeugt wird oder eine definierte Menge Konditioniermittel durch Elektrolyse in einem Abschnitt der Messeinrichtung erzeugt und der Messkammer zugeführt wird, eine Spannung zwischen Arbeitselektrode und Arbeitsreferenzelektrode angelegt wird und der Strom, der über die elektrische Verbindung von Arbeitselektrode und Arbeitsreferenzelektrode fließt, gemessen wird.

**10.** Verfahren nach einem der Ansprüche 1 bis 9, **dadurch gekennzeichnet, dass** das Konditioniermittel in der Messkammer durch eine Generatorelektrode erzeugt wird, wobei die Generatorelektrode, wenn die Arbeitselektrode als Kathode verwendet wird, als Anode betrieben wird, und wenn die Arbeitselektrode als Anode verwendet wird, als Kathode betrieben wird.

**11.** Amperometrischer Sensor zur Durchführung des in den vorausgehenden Ansprüchen definierten Verfahrens, wobei der Sensor mindestens eine in einer Messkammer zur Aufnahme des Elektrolyten angeordnete Arbeitselektrode, eine Arbeitsreferenzelektrode und eine selektiv durchlässige Membran, welche die Messkammer begrenzt, umfasst, **dadurch gekennzeichnet, dass** in der Messkammer eine Generatorelektrode und eine Generatorreferenzelektrode angeordnet sind, wobei eine Steuervorrichtung vorgesehen ist, mit der zwischen Arbeitselektrode und Arbeitsreferenzelektrode eine Messspannung angelegt werden kann und mit der zwischen Generatorelektrode und Generatorreferenzelektrode eine Generatorspannung angelegt wird, deren Betrag unterschiedlich oder gleich zu dem der Messspannung ist.

**12.** Amperometrischer Sensor nach Anspruch 11, **dadurch gekennzeichnet, dass** die Arbeitselektrode zumindest einen zylinderförmigen Abschnitt aufweist und die Generatorelektrode einen hohlzylinderförmigen Abschnitt aufweist, wobei die Arbeitselektrode mit ihrem zylinderförmigen Abschnitt innerhalb des hohlzylinderförmigen Abschnittes der Generatorelektrode angeordnet ist.

**13.** Amperometrischer Sensor nach Anspruch 11 oder 12, **dadurch gekennzeichnet, dass** die selektiv durchlässige Membran eine effektive Fläche hat, die kleiner oder gleich der der Membran zugewandten Fläche der Arbeitselektrode ist.

**14.** Messeinrichtung zur Durchführung des in den vorausgehenden Ansprüchen definierten Verfahrens, wobei die Einrichtung mindestens einen amperometrischen Sensor und eine Steuervorrichtung umfasst, **dadurch gekennzeichnet, dass** die Messeinrichtung mindestens eine in der Messeinrichtung angeordnete Generatorelektrode aufweist, wobei vorzugsweise die Messeinrichtung einen amperometrischen Sensor nach einem der Ansprüche 11 bis 13 aufweist.

**15.** Verwendung eines nach einem Verfahren der Ansprüche 1 bis 10 hergestellten Konditioniermittels zur Reinigung, Konditionierung, Kalibration und/oder Justage eines amperometrischen Sensors einer Messeinrichtung zur Bestimmung eines Inhaltsstoffes einer Probe.

**Figur 1**

**Figur 2**

**Figur 3**

**Figur 4**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CH 672845 A5
- EP 1452858 B1